(19) Europäisches Patentamt
European Patent Office
Office européen des brevets

(11) Veröffentlichungsnummer: **0 371 333 B1**

(12) # EUROPÄISCHE PATENTSCHRIFT

(45) Veröffentlichungstag der Patentschrift: **19.05.93**

(51) Int. Cl.$^{5}$: **A61K 7/02**, A61K 7/48, A61K 7/50

(21) Anmeldenummer: **89121185.6**

(22) Anmeldetag: **16.11.89**

Die Akte enthält technische Angaben, die nach dem Eingang der Anmeldung eingereicht wurden und die nicht in dieser Patentschrift enthalten sind.

(54) **Kosmetische Zubereitungen.**

(30) Priorität: **01.12.88 DE 3840525**

(43) Veröffentlichungstag der Anmeldung:
**06.06.90 Patentblatt 90/23**

(45) Bekanntmachung des Hinweises auf die Patenterteilung:
**19.05.93 Patentblatt 93/20**

(84) Benannte Vertragsstaaten:
**AT BE CH DE ES FR GB GR IT LI LU NL SE**

(56) Entgegenhaltungen:

| | |
|---|---|
| **EP-A- 21 827** | **CH-A- 622 181** |
| **DE-A- 1 617 694** | **DE-A- 2 107 429** |
| **DE-A- 2 646 435** | **DE-A- 2 729 888** |
| **DE-A- 2 856 716** | **DE-A- 2 900 370** |
| **DE-A- 3 124 822** | **DE-A- 3 207 004** |
| **FR-A- 2 097 333** | **FR-A- 2 589 356** |
| **US-A- 4 495 079** | |

(73) Patentinhaber: **Beiersdorf Aktiengesellschaft**
**Unnastrasse 48**
**W-2000 Hamburg 20(DE)**

(72) Erfinder: **Schönrock, Uwe, Dr.**
**Mittelstrasse 18b**
**W-2000 Norderstedt(DE)**
Erfinder: **Pape, Wolfgang, Dr.**
**Lydiastrasse 3**
**W-2000 Hamburg 70(DE)**
Erfinder: **Engel, Walter**
**Diesterwegstrasse 22**
**W-2080 Pinnenberg(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Die Erfindung betrifft kosmetische Zubereitungen, insbesondere Augen Make-up-Entferner.

Augen Make-up Entferner sind an sich bekannt. Üblicherweise handelt es sich dabei um Abmischungen kosmetischer Öle oder wäßrige Zubereitungen oberflächenaktiver Substanzen, deren Funktion darin besteht, den Make-up-Körper zu solubilisieren und von der Haut zu entfernen.

Derartige Make-up-Entferner werden angewandt, indem ein Wattebausch oder ein Tuch mit der kosmetischen Zubereitung getränkt und über Augenlider und Wimpern gerieben wird.

Wasserfestes Augen-Make-up, beispielsweise Mascara, ist mit Make-up-Entfernern auf wäßriger Basis nur mit speziellen Tensiden zufriedenstellend zu entfernen. Diese Tenside besitzen aber meist eine begrenzte physiologische Verträglichkeit. Bei einem Kontakt solcher Stoffe mit der Schleimhaut, insbesondere der Augenschleimhaut, führen diese Stoffe zu Reizungen, die sich beispielsweise in einer Rötung der Augen äußern. Reaktionen dieser Art sind typisch für tensidhaltige Produkte.

Zwar ist die Verwendung entzündungshemmender oder reizmindernder Stoffe bekannt und in vielen Formulierungen gebräuchlich. Auf diese Weise werden aber nur die Symptome unterdrückt und nicht die Ursache, nämlich der Reizstoff, beseitigt. In einem Alltagsprodukt wie einem Make-up-Entferner ist dies besonders bedenklich.

DE-OS 36 37 021 beschreibt schwach reizende Formulierungen, die unter anderem Wirkstoffe der allgemeinen Formeln

$R_1-(OCH_2CH_2)n-OH$ ($n=8-25$) und

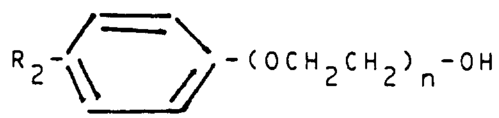

($n=10-40$) mit

$R_1=C_8-C_{18}$-Fettkette und

$R_2=C_8-C_{16}$ Alkylrest

enthalten.

Diese Formulierungen sind zwar schwach reizend, entfernen wasserfestes Makeup aber nicht befriedigend.

Aufgabe der vorliegenden Erfindung war somit, einen Make-up-Entferner auf wäßriger Basis zu entwickeln, der hervorragende Reinigungseigenschaften, aber ein niedriges Reizpotential besitzt. Er sollte imstande sein, selbst wasserfestes Makeup zu entfernen.

Es hat sich gezeigt, und darin liegt die Lösung der Aufgabe, daß

kosmetische Zusammensetzung en zur Verwendung als Augenmakeup-Entferner, enthaltend

a) einen oder mehrere grenzflächenaktive Stoffe der allgemeinen Formel $R-(O-CH_2-CH_2)_n-OH$, wobei R einen aliphatischen oder olefinischen Rest mit 8 - 20 Kohlenstoffatomen darstellt und der Ethoxylierungsgrad n eine Zahl von 15 - 120 bedeutet,

b) ein kosmetisches Öl oder ein Gemisch verschiedener kosmetischer Öle,

c) einen oder mehrere Lösungsvermittler, gewählt aus der Gruppe der Alkohole mit mindestens zwei Hydroxygruppen und gegebenenfalls Etherfunktionen,

d) 0,1 - 10 Gew.-% an Polyoxyethylen-Polyoxypropylen-Blockpolymeren mit mittleren Molekulargewichten von 2.000 - 3.000 und 2 - 15 Oxyethyleneinheiten und 15 - 30 Oxypropyleneinheiten je Block, bezogen auf die gesamte Zusammensetzung und

e) gegebenenfalls Hilfs- und Zusatzstoffe, Laurylsulfat ausgeschlossen,

in einem wäßrigen Medium, wobei diese Zusammensetzung keine Emulsion darstellt,

über die geforderten Eigenschaften verfügen.

Zwar ist aus der DE-OS 26 46 435 ein Verfahren zur Herstellung stabiler O/W-Emulsionen bekannt, enthaltend ein hydrophiles nichtionisches oberflächenaktives Mittel, ein wasserlösliches Lösungsmittel, ein Öl und Wasser. Die in dieser Schrift beschriebenen Zubereitungen stellen jedoch äußerst unansehnliche, kosmetisch wenig elegante Emulsionen und keine Lösungen dar. Diese Emulsionen sind zur Verwendung als Augenmakeup-Entferner ungeeignet.

In der US-Patentschrift 4,495,079 und in der Europäischen Patentanmeldungsschrift 213 827 werden Zubereitungen beschrieben, welche als oberflächenaktives Agens Natriumlaurylsulfat enthalten. Natriumlaurylsulfat aber besitzt ein hohes Reizpotential und ist zur Verwendung am Auge ungeeignet.

In Kenntnis dieser Schriften wäre es für den Fachmann nicht möglich gewesen, zur vorliegenden Erfindung zu gelangen.

Die erfindungsgemäßen Zusammensetzungen sind überraschenderweise imstande, selbst Mascara, eine stark deckende Schminke mit sehr schwach ausgeprägter Hydrophilie von Wimpern und Haut entfernen und besitzen ein sehr geringes Reizpotential für Schleimhäute.

Bevorzugt werden die grenzflächenaktiven Stoffe gewählt aus der Gruppe mit
R = Cetyl ($H_{33}C_{16}$),
R = Stearyl ($H_{37}C_{18}$),
R = Oleyl ($H_{33}C_{17}$),
R = Myristyl ($H_{29}C_{14}$),
R = Oleinyl ($H_{35}C_{18}$),
R = Isostearyl ($H_{31}C_{14}-CH(CH_3)CH_2$).
n nimmt dabei vorzugsweise Werte von 20 - 100 an.

Als besonders günstig hat sich erwiesen, die grenzflächenaktiven Substanzen aus der Gruppe
$H_{33}C_{16}-(O-CH_2-CH_2-)_{20}OH$,
$H_{37}C_{18}-(O-CH_2-CH_2)_{20}OH$
$H_{33}C_{17}-(O-Ch_2-CH_2)_{20}OH$ und
$H_{37}C_{18}-(O-CH_2-CH_2)_{100}OH$
$H_{31}C_{14}-CH(CH_3)CH_2-(O-CH_2-CH_2)_{20}-OH$
zu wählen.

Ganz besonders günstig sind Systeme, die zu etwa gleichen Teilen
$H_{33}C_{16}-(O-CH_2-CH_2-)_{20}OH$ und
$H_{37}C_{18}-(O-CH_2-CH_2-)_{100}OH$
oder
$H_{37}C_{18}-(O-CH_2-CH_2-)_{20}OH$ und
$H_{33}C_{17}-(O-CH_2-CH_2)_{20}OH$
oder
$H_{37}C_{18}-(O-CH_2-CH_2)_{20}OH$ und
$H_{37}C_{18}-(O-CH_2-CH_2)_{100}OH$
oder
$H_{37}C_{18}-(O-CH_2-CH_2)_{20}OH$ und
$H_{31}C_{14}-CH(CH_3)CH_2-(O-CH_2-CH_2)_{20}OH$
enthalten. Das Gewichtsverhältnis der beiden Einzelkomponenten zueinander liegt bevorzugt zwischen 1,5 : 1 und 1 : 1,5. Die Angaben für den Ethoxylierungsgrad n sind durchschnittliche Werte mit einer Schwankung von +/- 15 %.

Hydriertes, ethoxyliertes Ricinusöl ist bevorzugt als kosmetisches öl einzusetzen.

Wenn R einen olefinischen Rest darstellt, ist es günstig, ein Antioxidationsmittel in geeigneter Konzentration zuzufügen.

Vorzugsweise werden die Lösungsvermittler aus der Gruppe der Polyethylenglycole gewählt; vorteilhaft sind Polyethylenglycole eines mittleren Molekulargewichts von 300 - 700. Besonders vorteilhaft ist dabei ein mittleres Molekulargewicht von 350 - 500.

Es werden Zusammensetzungen mit günstigen Eigenschaften erhalten, wenn, bezogen auf die gesamte Zusammensetzung, 0,1 - 10 Gew.-% von Polyoxyethylen-Polyoxypropylen-Blockcopolymeren mit mittleren Molekulargewichten von 2.000 - 3.000 und 2 - 15 Oxyethyleneinheiten und 15 - 30 Oxypropyleneinheiten je Block beigefügt werden. Solche Blockcopolymere sind als Poloxamere bekannt.

Kosmetisches Öl und Lösungsvermittler sollten vorzugsweise in annähernd gleichen Gewichtsmengen zugegeben werden, als günstig haben sich Gewichtsverhältnisse von 1:2 bis 2:1 herausgestellt.

Es können die in der Kosmetik üblichen Hilfs- und Zusatzstoffe in die erfindungsgemäßen Zusammensetzungen eingearbeitet werden, beispielsweise
Antioxidationsmittel
Konservierungsmittel,
Parfum,
konsistenzgebende Stoffe,
Farbstoffe,
Puffersysteme.

Es hat sich herausgestellt, daß die erfindungsgemäßen Zusammensetzungen vorteilhaft auf einen pH-Wert zu bringen sind, welcher dem physiologischen pH-Wert der Tränenflüssigkeit entspricht, also 7,10 - 7,30. Besonders günstig sind Formulierungen, die zudem einen osmotischen Druck, entsprechend dem der

Tränenflüssigkeit, aufweisen.

Vorteilhaft enthalten die erfindungsgemäßen Zusammensetzungen

0,05 – 30 Gew.-% der beanspruchten grenzflächenaktiven Stoffe,
0,05 – 40 Gew.-% der beanspruchten kosmetischen Öle
0,05 – 30 Gew.-% der beanspruchten Lösungsvermittler,
bezogen auf die Gesamtzusammensetzung.

Besonders vorteilhaft sind Zusammensetzungen, die

1,0 – 15,0 Gew.-% der beanspruchten grenzflächenaktiven Stoffe,
1,0 – 17,5 Gew.-% der beanspruchten kosmetischen Öle,
1,0 – 12,0 Gew.-% der beanspruchten Lösungsvermittler,
bezogen auf die Gesamtzusammensetzung, enthalten.

Ganz besonders bevorzugt sind Systeme, enthaltend

1,2 – 5,3 Gew.-% Stoffe der beanspruchten grenzflächenaktiven Stoffe,
1,1 – 7,2 Gew.-% der beanspruchten kosmetischen Öle,
1,2 – 6,8 Gew.-% der beanspruchten Lösungsvermittler,
bezogen auf die Gesamtzusammensetzung.

Anhand der folgenden Beispiele sollen die erfindungsgemäßen Zusammensetzungen beispielhaft erläutert werden, ohne daß damit eine Beschränkung auf diese Beispiele beabsichtigt ist.

Die Verträglichkeit, insbesondere die Schleimhautverträglichkeit, wurde mit sogenannten in vitro Ersatzmethoden zur Abschätzung der Augenschleimhautverträglichkeit (OECD-Guideline for Testing of Chemicals No. 405, v. 24. Februar 1987) durchgeführt.

1. Als grundlegender Test wurde das RBC-Test-System (nach W. Pape & U. Hoppe, 1988, 2nd World Surfactant Congress, Paris, Proceedings, Vol. IV p. 415 ff.) zur Abschätzung des Reizpotentials angewandt.
2. Darüberhinaus wurde eine sensiblere Modifikation des Tests an der Chorionallantois-Membran (CAM) bebrüteter Hühnereier (Lohmann's selected white Leghorn) nach K. Künstler et al. (15th IFCC-Kongress, Vol. 1., pp. 11-24, Barcelona 1986) zur Beurteilung der Irritanz durchgeführt. Die Veränderung der Methode besteht im wesentlichen darin, daß ein Stereomikroskop zur empfindlicheren Beurteilung morphologischer Veränderungen an der CAM eingesetzt wurde.

Ergebnisse

1. Die Formulierungen wurden im Sinne der Beurteilung am Auge nach Draize et al. (OECD-Guideline 405) im RBC-Test-System als "nicht irritant" erkannt. (Tabelle 1).
2. Die sehr schwachen Reizungen lassen sich nach der Reaktionszeitmethode differenzieren und ergeben für die Formulierungen der Beispiele 1 – 4 Werte zwischen 40 – 182 (Tabelle 1). Eine Formulierung gemäß DE-OS 36 37 021 wurde unter den gleichen Bedingungen getestet.

Tabelle 1

| Beispiel | 1 | 2 | 3 | 4 | Vergleichssubstanz gemäß DE-OS 36 37 021 Beispiel 1 |
|---|---|---|---|---|---|
| GFS, R = Oleyl, n = 20 | - | 1,0 g | - | - | |
| GFS, R = Cetyl, n = 20 | 1,0 g | - | - | - | |
| GFS, R = Stearyl, n = 20 | - | 1,0 g | 1,0 g | 1,0 g | |
| GFS, R = Stearyl, N = 100 | 1,0 g | - | 1,0 g | - | |
| GFS, R = Isostearyl, n = 20 | - | - | - | 1,0 g | |
| Polyethylenglycol, MW=400/500: | 2,0 g | 2,0 g | 2,0 g | 2,0 g | |
| Poloxamer 105 | - | 5,0 g | - | 5,0 g | |
| Rizinusöl, hydriert und ethoxyliert: | 3,0 g | 3,0 g | 3,0 g | 3,0 g | |
| Stabilisatoren: | \|---------n.B.--------\| | | | | |
| Konservierungsmittel: | \|---------n.B.--------\| | | | | |
| Parfüm: | \|---------n.B.--------\| | | | | |
| Farbe: | \|---------n.B.--------\| | | | | |
| Wasser: | jeweils auf 100 g | | | | |
| pH-Wert: | 7,1 | 7,1 | 7,1 | 7,1 | |
| RBC-Test: | \|--------nicht irritant-------------------------\| | | | | |
| HET-CAM Reizwerte: | 182,0 | 41,4 | 145,0 | 129,4 | 287,0 |

GFS: erfindungsgemäße Grenzflächenaktive Substanz.

Poloxymer 105: MW = 1.900, 11 Oxyethyleneinheiten und 16 Oxypropyleneinheiten je Block

Beispiel 5

Blondes Haar wurde auf Wimpernlänge zurechtgeschnitten und auf Glasplatten geklebt. Jede Probe wurde zweimal mit handelsüblichem wasserfestem Mascara bestrichen. Die so vorbereiteten Proben wurden mit Wattebäuschen bestrichen, welche mit Formulierungen gemäß den Beispielen 1, 2, 3 und 4 getränkt worden waren. Die Beurteilung der Formulierung erfolgte optisch anhand der Entfärbung des Haars. Nach achtmaligem Bestreichen der Haarproben mit den erfindungsgemäßen Formulierungen war das Make-up praktisch völlig entfernt. Handelsübliche Formulierungen auf der Basis von Amphotensiden in wäßriger

Lösung dienten als Vergleich. Die entsprechend behandelten Haarproben waren erst nach zwölfmaligem Bestreichen frei von Make-up-Resten.

## Patentansprüche

1. Kosmetische Zusammensetzung zur Verwendung als Augenmakeup-Entferner, enthaltend
   a) einen oder mehrere grenzflächenaktive Stoffe der allgemeinen Formel $R-(O-CH_2-CH_2)_n-OH$, wobei R einen aliphatischen oder olefinischen Rest mit 8 - 20 Kohlenstoffatomen darstellt und der Ethoxylierungsgrad n eine Zahl von 15 - 120 bedeutet,
   b) ein kosmetisches Öl oder ein Gemisch verschiedener kosmetischer Öle,
   c) einen oder mehrere Lösungsvermittler, gewählt aus der Gruppe der Alkohole mit mindestens zwei Hydroxygruppen und gegebenenfalls Etherfunktionen,
   d) 0,1 - 10 Gew.-% an Polyoxyethylen-Polyoxypropylen-Blockpolymeren mit mittleren Molekulargewichten von 2.000 - 3.000 und 2 - 15 Oxyethyleneinheiten und 15 - 30 Oxypropyleneinheiten je Block, bezogen auf die gesamte Zusammensetzung, und
   e) gegebenenfalls Hilfs- und Zusatzstoffe, Laurylsulfat ausgeschlossen,

   in einem wäßrigen Medium, wobei diese Zusammensetzung keine Emulsion darstellt.

2. Zusammensetzung nach Anspruch 1, dadurch gekennzeichnet, daß der chemische Rest R aus der Gruppe Cetyl-, Stearyl-, Oleyl-, Myristyl-, Oleinyl-, Isostearylgewählt ist und n eine Zahl von 20 - 100 darstellt.

3. Zusammensetzung nach Anspruch 1 oder 2, dadurch gekennzeichnet, daß die grenzflächenaktiven Stoffe Gemische aus
   Cetyl$-(O-CH_2-CH_2)_{20}OH$ und
   Stearyl$-(O-CH_2-CH_2)_{100}OH$
   oder
   Stearyl$-(O-CH_2-CH_2)_{20}OH$ und
   Oleyl$-(O-CH_2-CH_2)_{20}OH$
   oder
   Stearyl$-(O-CH_2-CH_2)_{20}OH$ und
   Stearyl$-(O-CH_2-CH_2)_{100}OH$
   oder
   Stearyl$-(O-CH_2-CH_2)_{20}OH$ und
   Isostearyl$-(O-CH_2-CH_2)_{20}OH$
   darstellen, wobei das Gewichtsverhältnis der Komponenten zueinander im Bereich von 1 : 1,5 bis 1,5 : 1 liegt.

4. Zusammensetzung nach einem der Ansprüche 1 - 3, dadurch gekennzeichnet, daß der oder die Lösungsvermittler aus der Gruppe der Polyethylenglylcole mit mittleren Molekulargewichten von 300 - 500 Dalton gewählt werden.

5. Zusammensetzung nach einem der Ansprüche 1 - 4, dadurch gekennzeichnet, daß das kosmetische Öl hydriertes und ethoxyliertes Ricinusöl ist.

6. Zusammensetzung nach einem der vorangehenden Ansprüche, enthaltend
   0,05 - 30,0 Gew.-%, vorteilhaft 1,0 - 15,0 Gew.-%, besonders vorteilhaft 1,2 - 6,3 Gew.-% an grenzflächenaktiven Stoffen,
   0,05 - 40,0 Gew.-%, vorteilhaft 1,0 - 17,5 Gew.-%, besonders vorteilhaft 1,1 - 7,2 Gew.-% eines oder mehrerer kosmetischer Öle und
   0,05 - 30,0 Gew.-%, vorteilhaft 1,0 - 14,0 Gew.-%, besonders vorteilhaft 1,2 - 8,8 Gew.-% eines oder mehrerer Lösungsvermittler, jeweils bezogen auf die gesamte Zusammensetzung.

7. Zusammensetzung nach einem der vorangehenden Ansprüche, durch einen pH-Wert von 7,10 - 7,30 gekennzeichnet.

## Claims

1. Cosmetic composition for use as eye make-up remover which contains
   a) one or more surface-active substances of the general formula $R-(O-CH_2-CH_2)_n-OH$, where R represents an aliphatic or olefinic radical with 8 - 20 carbon atoms and the degree of ethoxylation n denotes a number from 15 - 120,
   b) a cosmetic oil or a mixture of a range of cosmetic oils,
   c) one or more solubilisers selected from the group of the alcohols having at least two hydroxyl groups and, if appropriate, ether functions
   d) 0.1 - 10% by weight of polyoxyethylene/polyoxypropylene block copolymers having mean molecular weights of 2000 - 3000 and 2 - 15 oxyethylene units and 15 - 30 oxypropylene units per block, based on the entire composition, and
   e) if appropriate adjuvants and additives, with the exclusion of lauryl sulphate,

   in an aqueous medium, but without this composition representing an emulsion.

2. Composition according to Claim 1, characterised in that the chemical radical R is selected from amongst the grow cetyl-[sic], stearyl-[sic], oleyl-[sic], myristyl-[sic], oleinyl-[sic], isostearyl-[sic] and n represents a number from 20 - 100.

3. Composition according to Claim 1 or 2, characterised in that the surface-active substances represent mixtures of
   cetyl$-(O-CH_2-CH_2)_{20}OH$ and
   stearyl$-(O-CH_2-CH_2)_{100}OH$
   or
   stearyl$-(O-CH_2-CH_2)_{20}OH$ and
   oleyl$-(O-CH_2-CH_2)_{20}OH$
   or
   stearyl$-(O-CH_2-CH_2)_{20}OH$ and
   stearyl$-(O-CH_2-CH_2)_{100}OH$
   or
   stearyl$-(O-CH_2-CH_2)_{20}OH$ and
   isostearyl$-(O-CH_2-CH_2)_{20}OH$,
   the ratio by weight of the components to each other being in the range from 1 : 1.5 to 1.5 : 1.

4. Composition according to one of Claims 1 - 3, characterised in that the solubiliser, or solubilisers, are selected from the group of the polyethylene glylcols [sic] having mean molecular weights from 300 - 500 Dalton.

5. Composition according to one of Claims 1 - 4, characterised in that the cosmetic oil is hydrogenated and ethoxylated castor oil.

6. Composition according to one of the preceding claims, containing
   0.05 - 30.0% by weight, advantageously 1.0 - 15.0% by weight, particularly advantageously 1.2 - 6.3% by weight of surface-active substances,
   0.05 - 40.0% by weight, advantageously 1.0 - 17.5% by weight, particularly advantageously 1.1 - 7.2% by weight of one or more cosmetic oils and
   0.05 - 30.0% by weight, advantageously 1.0 - 14.0% by weight, particularly advantageously 1.2 - 8.8% by weight, of one or more solubilisers, in each case based on the total composition.

7. Composition according to one of the preceding claims, characterised in that it has a pH of 7.10 - 7.30.

## Revendications

1. Composition cosmétique à utiliser en tant que démaquillant pour les yeux, comprenant
   a) une ou plusieurs substances tensioactives de formule générale $R-(O-CH_2-CH_2)_n-OH$, dans laquelle R représente un radical aliphatique ou oléfinique ayant de 8 à 20 atomes de carbone et dans laquelle le degré d'éthoxylation n représente un chiffre de 15 à 120,
   b) une huile cosmétique ou un mélange de différentes huiles cosmétiques,

c) un ou plusieurs agents de solubilisation, choisi(s) parmi le groupe des alcools ayant au moins deux groupes hydroxyles et éventuellement des fonctions éther,
d) de 0,1 à 10 % en poids de polymères en bloc polyoxyéthylène – polyoxypropylène ayant des poids moléculaires moyens de 2000 à 3000 et ayant de 2 à 15 unités oxyéthylène et de 15 à 30 unités oxypropylène par bloc, sur la base de la composition globale, et
e) éventuellement des adjuvants et additifs, à l'exception du laurylsulfate,
dans un milieu aqueux, cette composition ne constituant pas une émulsion.

**2.** Composition selon la revendication 1, caractérisée en ce que le radical chimique R est choisi parmi le groupe cétyle, stéaryle, héptadécyle, myristyle, oléyle, isostéaryle et que n représente un chiffre de 20 à 100.

**3.** Composition selon la revendication 1 ou 2, caractérisée en ce que les substances tensioactives représentent des mélanges de
cétyl – $(O-CH_2-CH_2)_{20}OH$ et
stéaryl – $(O-CH_2-CH_2)_{100}OH$
ou
stéaryl – $(O-CH_2-CH_2)_{20}OH$ et
hétadécyle – $(O-CH_2-CH_2)_{20}OH$
ou
stéaryl – $(O-CH_2-CH_2)_{20}OH$ et
stéaryl – $(O-CH_2-CH_2)_{100}OH$
ou
stéaryl – $(O-CH_2-CH_2)_{20}OH$ et
isostéaryl – $(O-CH_2-CH_2)_{20}OH$,
le rapport en poids des composants l'un par rapport à l'autre étant dans le domaine de 1 : 1,5 à 1,5 : 1.

**4.** Composition selon l'une des revendications 1 à 3, caractérisée en ce que le ou les agents de solubilisation sont choisis parmi le groupe des polyéthylène – glycols ayant des poids moléculaires moyens de 300 à 500 daltons.

**5.** Composition selon l'une des revendications 1 à 4, caractérisée en ce que l'huile cosmétique est une huile de ricin hydrogénée et éthoxylée.

**6.** Composition selon l'une des revendications précédentes, comprenant
de 0,05 à 30,0 % en poids, avantageusement de 1,0 à 15,0 % en poids, plus avantageusement encore de 1,2 à 6,3 % en poids de substances tensioactives,
de 0,05 à 40,0 % en poids, avantageusement de 1,0 à 17,5 % en poids, plus avantageusement encore de 1,1 à 7,2 % en poids de l'une ou de plusieurs huiles cosmétiques et
de 0,05 à 30,0 % en poids, avantageusement de 1,0 à 14,0 % en poids, plus avantageusement encore de 1,2 à 8,8 % en poids de l'un ou de plusieurs agents de solubilisation, à chaque fois sur la base de la composition globale.

**7.** Composition selon l'une quelconque des revendications précédentes, caractérisée par une valeur pH de 7,10 à 7,30.